# EUROPEAN PATENT APPLICATION

(11) **EP 2 375 353 A1**
(43) Date of publication of application: **12.10.2011**
(21) Application number: 11159878.5
(22) Date of filing: 25.03.2011
(51) Int. Cl.: G06F 19/00, G06Q 10/00

(54) **Systems and methods for redacting sensitive data entries**

(30) Priority: 25.03.2010 US 317478 P
(71) Applicant: RL Solutions, Toronto, ON M5T 2T3 (CA)
(72) Inventor: Hung, Colin, Markham Ontario L6C 2K1 (CA); Leung, Cecil, Toronto Ontario M4M 3P4 (CA); Han, Zheng, Toronto Ontario M1L 0A4 (CA)
(74) Representative: Scaddan, Gareth Casey

(57) **Abstract**

Systems and methods for redacting sensitive data entries are described. A command to a document is received, where the document comprises data entries, wherein each data entry comprises text-based content. One or more sensitive data entries in the document are identified. A link between each identified sensitive data entry and a descriptor label is defined, and the link and the descriptor label are stored in a repository. A redacted document is generated by, for each identified sensitive data entry, replacing the text-based content with the associated descriptor label.

## Description

### Field of the Invention

The present invention relates to systems and methods for redacting sensitive data entries. Embodiments of the present invention relate in particular to systems and methods for redacting sensitive data entries in a document to generate a redacted document.

### Background of the Invention

People share documents with other people. A document may contain sensitive information that the person may not want to share. Sensitive information may be private information, personal information, confidential information or other information unsuited for sharing. For example, sensitive information may include a name, a physical address, a bank account, medical information, social security numbers, driver's license numbers, telephone numbers, e-mail addresses, a password, a user name, and employment information.

To protect the sensitive information before sharing the document, a person can redact the sensitive information. For example, a person can print a copy of the document and manually black out the text using a black marker. As another example, some electronic systems provide a black marker tool that allows a user to manually black out the text in an electronic version of the document. A person can also edit an electronic copy of the document by deleting the sensitive text. These known systems and methods require a person to manually review all information in the document and individually black out all pieces of sensitive information. Blacking out sensitive information in this fashion can be time consuming and prone to human error. Deleting the sensitive text does not visually indicate that the sensitive information has been redacted, and is also time consuming and prone to human error. Redacting by blacking out sensitive information does not provide any indication as to the type of information that is being redacted. This may cause the non-redacted portions of the redacted document to be unintelligible to the recipient of the redacted document.

### Summary of the Invention

Various respective aspects and features of the invention are defined in the appended claims. Combinations of features from the dependent claims may be combined with features of the independent claims as appropriate and not merely as explicitly set out in the claims.

Some embodiments of the invention provide a method for redacting sensitive data entries, wherein the method is implemented on a processor having access to a memory in which instructions are stored, the instructions being executable to configure the processor to perform operations comprising:
receiving a command to generate a document, wherein the document comprises data entries, wherein each data entry comprises text-based content;
identifying at least one sensitive data entry in the document;
defining a link between each identified sensitive data entry and a descriptor label;
storing the link and the descriptor label in a repository; and
generating a redacted document by, for each identified sensitive data entry, replacing the text-based content with the associated descriptor label.

Some embodiments of the invention provide the method further comprising:
receiving a command to redact at least one sensitive data entry in the document;
   and
generating the redacted document by, for each of the at least one sensitive data entries in the command to redact, replacing the text-based content with the associated descriptor label.

Some embodiments of the invention provide the method further comprising:
receiving a single command to redact all sensitive data entries in the document;
identifying all sensitive data entries in the document;
generating the redacted document by, for each sensitive data entry in the document, replacing the text-based content with the associated descriptor label.

Some embodiments of the invention provide the method, wherein the command to generate the document comprises a user identifier, and wherein at least one identified sensitive data entry in the document is identified based on the user identifier. Some embodiments of the invention provide the method further comprising receiving a command to export the document; and exporting the redacted document.

Some embodiments of the invention provide the method, wherein the command to export comprises a recipient identifier, and wherein at least one identified sensitive data entry in the document is identified based on the recipient identifier

Some embodiments of the invention provide the method, wherein exporting is selected from the group consisting of: printing, saving, transmitting and emailing.

Some embodiments of the invention provide the method wherein each sensitive data entry is selectable, and wherein the method further comprises:
receiving a selected sensitive data entry;
receiving a descriptor label; and
defining a link between the selected sensitive data entry and the received descriptor label.

Some embodiments of the invention provide the method wherein each sensitive data entry is selectable, and wherein the method further comprises:
receiving a selected sensitive data entry;
providing a set of descriptor labels, wherein each descriptor label is selectable;
receiving a selected descriptor label; and
defining a link between the selected sensitive data entry and the selected descriptor label.

Some embodiments of the invention provide the method wherein determining whether the document contains one or more sensitive data entries comprises:
receiving sensitive text-based content;
determining whether a data entry in the document comprises the sensitive text-based content; and
upon determining that the data entry in the document comprises the sensitive text-based content, determining that the data entry is a sensitive data entry.

Some embodiments of the invention provide the method wherein determining whether the document comprises one or more sensitive data entries comprises:
associating at least one data entry in the document with a data type;
defining at least one data type as a sensitive data type;
determining, for each of the at least one data entry in the document, whether the associated data type is a sensitive data type;
upon determining that the associated data type is a sensitive data type, determining that the data entry is a sensitive data entry.

Some embodiments of the invention provide the method wherein the identified sensitive data entry comprises sensitive text-based content; wherein at least one additional data entry in the document comprises the sensitive text-based content; and wherein the method further comprises:
identifying the at least one additional data entry as a sensitive data entry using the sensitive text-based content;

Some embodiments of the invention provide the method wherein at least one descriptor label is selected from a set of predetermined descriptor labels.

Some embodiments of the invention provide the method wherein at least one descriptor label is a user-defined descriptor label; wherein the method further comprises receiving the user-defined descriptor label.

Some embodiments of the invention provide the method wherein defining a link between each sensitive data entry and a descriptor label further comprises:
defining, for each sensitive data entry, a link between the sensitive data entry
   and a data type; and
defining a link between each data type and a descriptor label.

Some embodiments of the invention provide the method wherein the at least one data entry in the document comprises a form field data value; and wherein the method further comprises:
defining a link between the form field data value and a corresponding form field object, wherein the field object is configured to define a form field, wherein the form field is configured to receive the form field data value;
defining attributes for the form field object, wherein the attributes comprise a sensitive data determination attribute and a caption attribute;
determining that the at least one data entry in the document is a sensitive data entry based on the sensitive data determination attribute of the corresponding form field object; and
defining the linked descriptor label using the caption attribute of the corresponding form field object.

Some embodiments of the invention provide the method further comprising:
providing the document using a mark up language, wherein the document comprises a mark up language attribute tag for each sensitive data entry in the document;
identifying a sensitive data entry using the mark up language attribute tag in the document; and
generating the redacted document using the mark up language attribute tag for the sensitive data entry.

Some embodiments of the invention provide the method wherein the document selected from the group consisting of a picture, a form, a field, a report, a memo, and attachment to a form.

Some embodiments of the invention provide the method wherein the descriptor label is any html enabled object.

Some embodiments of the invention provide a method comprising:
receiving a command to generate a document, wherein the document comprises data entries, wherein each data entry comprises text-based content;
receiving a command to export the document;
receiving a single command to redact all sensitive data entries in the document;
identifying all sensitive data entries in the document;
generating a redacted document by, for each sensitive data entry in the document, replacing the text-based content with the associated descriptor label; and exporting the redacted document.

Some embodiments of the invention provide a computing system for redacting sensitive data entries comprising:
at least one processor and at least one memory, wherein the processor is configured to execute instructions stored in the memory to provide:
   a user interface component configured to:
receive a command to generate a document, wherein the document comprises data entries, wherein each data entry comprises text-based content;
provide a redacted document;
redaction module configured to:
   identify at least one sensitive data entry in the document;
   define a link between each identified sensitive data entry and a descriptor label;
   store the link and the descriptor label in a repository; and
   generate a redacted document by, for each identified sensitive data entry, replacing the text-based content with the associated descriptor label.

Some embodiments of the invention provide the system wherein the user interface component is further configured to receive a command to redact at least one sensitive data entry in the document; and wherein the redaction module is further configured to generate the redacted document by, for each of the at least one sensitive data entries in the command to redact, replacing the text-based content with the associated descriptor label.

Some embodiments of the invention provide the system wherein the user interface component is further configured to receive a single command to redact all sensitive data entries in the document; and wherein the redaction module is further configured to identify all sensitive data entries in the document, and to generate the redacted document by, for each of the at least one sensitive data entries in the document, replacing the text-based content with the associated descriptor label.

Some embodiments of the invention provide the system wherein the command to generate the document comprises a user identifier; and wherein the redaction module is further configured to identify at least one sensitive data entry in the document based on the user identifier.

Some embodiments of the invention provide the system wherein the user interface component is further configured to receive a command to export the document; and the system further comprises an export module configured to export the redacted document.

Some embodiments of the invention provide the system wherein the command to export comprises a recipient identifier, and wherein the redaction module is further configure to identify at least one sensitive data entry in the document based on the recipient identifier.

Some embodiments of the invention provide the system wherein the export module is configured to export the redacted document from the group consisting of: print, save, transmit and email.

Some embodiments of the invention provide the system wherein the user interface component is configured to provide each sensitive data entry as selectable, and wherein the redaction component is further configured to:
receive a selected sensitive data entry;
receive a descriptor label; and
define a link between the selected sensitive data entry and the received descriptor label.

Some embodiments of the invention provide the system wherein the user interface component is configured to provide each sensitive data entry as selectable and wherein the redaction component is further configured to:
receive a selected sensitive data entry;
provide a set of descriptor labels, wherein each descriptor label is selectable;
receive a selected descriptor label; and
define a link between the selected sensitive data entry and the selected descriptor label.

Some embodiments of the invention provide the system wherein the redaction module is configured to:
associate at least one data entry in the document with a data type;
define at least one data type as a sensitive data type;
determine that at least one data entry in the document is associated with a sensitive data type;
determine that the at least one data entry is a sensitive data entry.

Some embodiments of the invention provide the system wherein the redaction module is configured to: identify the at least one additional data entry as a sensitive data entry using sensitive text-based content.

Some embodiments of the invention provide the system wherein the redaction module is configured to provide a set of predetermined descriptor labels.

Some embodiments of the invention provide the system wherein the redaction module is configured to receive at least one user-defined descriptor label.

Some embodiments of the invention provide the system wherein the redaction module is configured to define for each sensitive data entry, a link between the sensitive data entry and a data type; and define a link between each data type and a descriptor label.

Some embodiments of the invention provide the system further comprising a form engine configured to provide a form and receive form field data values at the form; and define attributes for field objects, wherein the attributes comprise a sensitive data determination attribute and a caption attribute;
and wherein the redaction module is further configured to:
define a link between the form data value and a corresponding form object, wherein the field object is configured to define a form field, wherein the form field is configured to receive the form field data value;
determine that the at least one data entry in the document is a sensitive data entry based on the sensitive data determination attribute of the corresponding form field object; and
define the linked descriptor label using the caption attribute of the corresponding form field object.

Some embodiments of the invention provide the system wherein the user interface component is configured to provide the document using a mark up language, wherein the document comprises a mark up language attribute tag for each sensitive data entry in the document;
and wherein the redaction module is configured to identify a sensitive data entry using the mark up language attribute tag in the document;
and wherein the user interface component is configured to generate the redacted document using the mark up language attribute tag for the sensitive data entry.

Some embodiments of the invention provide a non-transitory computer-readable medium upon which a plurality of instructions are stored, the instructions for performing the steps of the method described herein.

### Brief Description of the Drawings

Embodiments of the invention will now be described with reference to the accompanying drawings, throughout which like parts are referred to by like references, and in which:
Figure 1 is a block diagram of a system for redacting sensitive data entries in accordance with an example embodiment;
Figure 2 is a block diagram illustrating the components of a workstation of a system for redacting sensitive data entries in accordance with an example embodiment;
Figure 3 is a flow diagram of a method for redacting sensitive data entries in accordance with an example embodiment;
Figure 4 is a screen shot diagram of a user interface component for receiving a command to redact a sensitive data entry in a document in accordance with an example embodiment;
Figure 5 is screen shot diagram of a user interface component for providing a set of selectable descriptor labels in accordance with an example embodiment;
Figure 6 is a screen shot diagram of a user interface component for receiving a single command to redact all sensitive data entries in a document in accordance with an example embodiment;
Figure 7 is a screen shot diagram of a user interface component for receiving a single command to redact all sensitive data entries in a document in accordance with an example embodiment;
Figure 8 is a screen shot diagram of a user interface component for receiving a single command to redact all sensitive data entries in a document in accordance with an example embodiment;
Figure 9 is a screen shot diagram of a user interface component for defining one or more data types as a sensitive data type in a document in accordance with an example embodiment;
Figure 10 is a screen shot diagram of a user interface component for defining a data type as a sensitive data type in accordance with an example embodiment;
Figure 11 is a screen shot diagram of a user interface component illustrating the link between a sensitive data type and the descriptor label in accordance with an example embodiment; and
Figure 12 is a screen shot diagram of a user interface component for receiving sensitive text and a descriptor label for a sensitive data type in a document in accordance with an example embodiment.

The skilled person in the art will understand that the drawings, described below, are for illustration purposes only. The drawings are not intended to limit the scope of the applicants' teachings in anyway. Also, it will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### Description of the Example Embodiments

It will be appreciated that numerous specific details are set forth in order to provide a thorough understanding of the exemplary embodiments described herein. However, it will be understood by those of ordinary skill in the art that the embodiments described herein may be practiced without these specific details. In other instances, well-known methods, procedures and components have not been described in detail so as not to obscure the embodiments described herein. Furthermore, this description is not to be considered as limiting the scope of the embodiments described herein in any way, but rather as merely describing the implementation of the various embodiments described herein.

The embodiments of the systems and methods described herein may be implemented in hardware or software, or a combination of both. However, preferably, these embodiments are implemented in computer programs executing on programmable computers each comprising at least one processor, a data storage system (including volatile and non-volatile memory and/or storage elements), and at least one communication interface. For example and without limitation, the programmable computers may be a server, network appliance, set-top box, embedded device, computer expansion module, personal computer, laptop, personal data assistant, or mobile device. Program code is applied to input data to perform the functions described herein and generate output information. The output information is applied to one or more output devices, in known fashion. In some embodiments, the communication interface may be a network communication interface. In embodiments where elements of the invention are combined, the communication interface may be a software communication interface, such as those for inter-process communication (IPC). In still other embodiments, there may be a combination of communication interfaces.

Each program is preferably implemented in a high level procedural or object oriented programming and/or scripting language to communicate with a computer system. However, the programs can be implemented in assembly or machine language, if desired. In any case, the language may be a compiled or interpreted language. Each such computer program is preferably stored on a storage media or a device (e.g. ROM or magnetic diskette) readable by a general or especial purpose programmable computer, for configuring and operating the computer when the storage media or device is read by the computer to perform the procedures described herein. The inventive system may also be considered to be implemented as a computer-readable storage medium, configured with a computer program, where the storage medium so configured causes a computer to operate in a specific and predefined manner to perform the functions described herein.

Furthermore, the system, processes and methods of the described embodiments are capable of being distributed in a computer program product comprising a physical computer readable medium that bears computer usable instructions for one or more processors. The medium may be provided in various forms, including one or more diskettes, compact disks, tapes, chips, magnetic and electronic storage media, and the like. The computer useable instructions may also be in various forms, including compiled and non-compiled code.

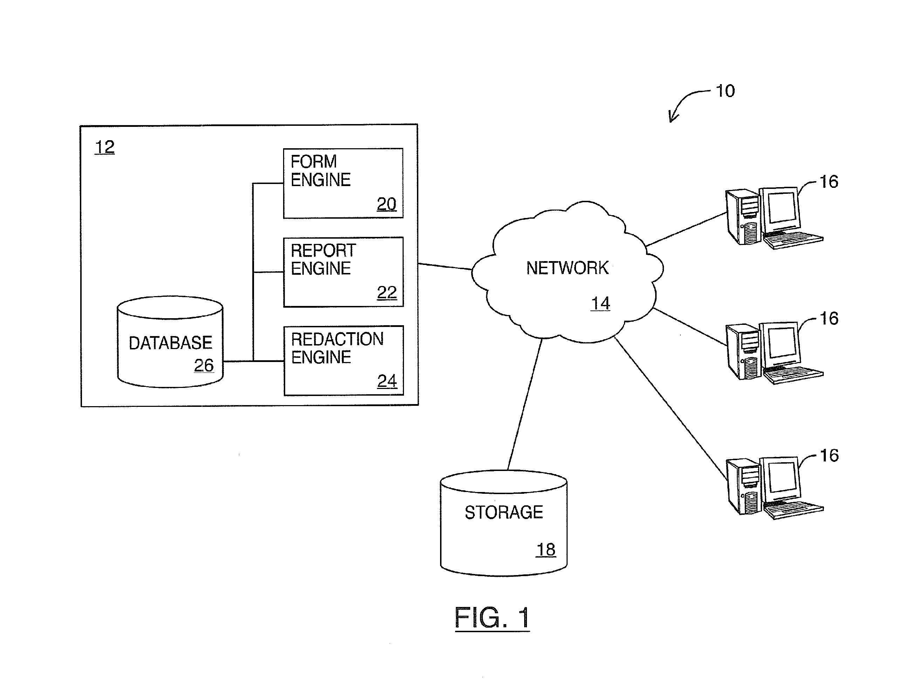

Figure 1 is a block diagram of a system 10 for redacting sensitive data entries in accordance with an example embodiment. System 10 includes a data system 12, workstations 16, and storage 18, connected via network 14.

Network 14 may be any network capable of carrying data, including the Internet, Ethernet, plain old telephone service (POTS) line, public switch telephone network (PSTN), integrated services digital network (ISDN), digital subscriber line (DSL), coaxial cable, fiber optics, satellite, mobile, wireless (e.g. Wi-Fi, WiMAX), SS7 signaling network, fixed line, local area network, wide area network, and others, including any combination of these, capable of interfacing with, and enabling communication between, data system 12, workstations 16, and storage 18.

Data system 12, workstations 16, and storage 18 may be connected to network 14 through a firewall (not shown), which is a device, set of devices or software that inspects network traffic passing through it, and denies or permits passage based on a set of rules and other criteria. Firewall may be adapted to permit, deny, encrypt, decrypt, or proxy all computer traffic based upon a set of rules and other criteria. For example, firewall may be a network layer firewall, an application layer firewall, a proxy server, or a firewall with network address translation functionality. Firewall provides a security mechanism to protect data stored in data system 12.

Storage 18 is a hardware and software storage system, which may include volatile and non-volatile memory and/or storage elements. Although shown connected to data system 12 and workstation 16 via network, storage 18 may be internal to workstation 16 and data system 12.

As an illustrative example, data system 12 will be described herein as a health care incident management system 12. However, data system 12 may be implemented in a wide variety of systems that collect, manage and export sensitive, private, confidential or personal data.

Generally, health care incident management system 12 is operable to manage a large amount of health care incident data. Health care incident data may include information that a user is permitted to share with others as well as personal and confidential information that a user may not be permitted to share.

Health care incident management system 12 is operable to receive data relating to health care incidents via forms displayed on workstations 16, for example, and store the received data in a database 26. Health care incident management system 12 is further operable to generate reports using the stored data, such as incident file summaries and patient records. A user may request a copy of the report to share but may want to redact the sensitive information before sharing the report. Health care incident management system 12 is operable to generate a redacted document suitable for sharing with the intended recipient. Health care incident management system 12 generates the redacted document by replacing the sensitive information with descriptor labels. The user can initiate a command to redact all sensitive data entries in a document, all sensitive data entries of a specific data type, or an individual sensitive data entry in a document. The command to redact may be automatically initiated when the user requests a document for sharing by print, email, file transmission, and the like.

In an example embodiment, health care incident management system 12 has a processor and a memory storing instructions, the instructions being executable to configure the processor to provide a number of functional elements including: a form engine 20, a report engine 22, a redaction module 24, and a database 26.

Form engine 20 is operable to generate a form, which is a collection of form fields operable to receive field value data. For example, the form may relate to a health care incident involving a patient and the form fields are operable to receive field values pertaining to the health care incident, such as the patient information, a description of the incident, names of other people involved in the incident, the date of the incident, and the time of the incident, for example. Data system 12 is operable to store received field values in database 26 (or storage 18) as field values. Form engine is operable to generate a form of form fields using field objects stored in the database 26 (or storage 18). Each form field is an instance of a specific field object, which defines a set of attributes for a form field.

Report engine 22 is operable to generate reports using data in database 26, including the stored field values. A report is a document that may include text, tables, figures, pictures, attachments, abstracts, summaries, appendices, footnotes, hyperlinks, charts, graphs and the like. For example, the report may be a medical incident report outlining all health care incidents involving a specific patient. As another example, a report may provide a summary of field value data received in relation to a specific health care incident.

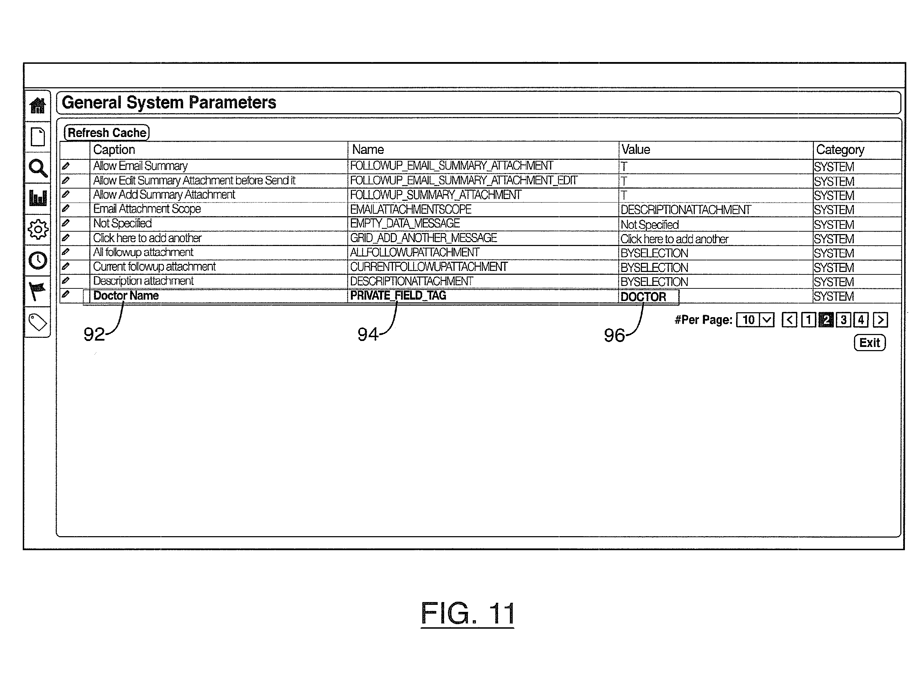

Redaction module 24 is operable to determine whether a document includes sensitive data entries and to define a link between each sensitive data entry and a descriptor label. For example, if the document is a medical incident report, a sensitive data entry may include text-based content such as a doctor's name, and the descriptor label may be "DOCTOR". Figure 11 is a screen shot diagram of a user interface component illustrating the link between a sensitive data type 92 (DOCTOR NAME), a descriptor label name 94 (e.g. PRIVATE FIELD TAG), and a descriptor label 96 (DOCTOR) in accordance with an example embodiment. An administrative user may access data system 12 via workstation 16 to modify the descriptor label 96 text and otherwise configure the descriptor label 96.

Redaction module 24 is further operable to generate a redacted document by replacing sensitive data in the document with linked descriptor label(s). For example, the document may include the following memo that includes sensitive data entries:

| Incident Summary: Roberta Fuentes, 123444 |
|---|
| Cecil Leung submitted a diabetic diagnosis for Bonnie Smith, 123453 with instructions for Colin Hung to provide insulin three times daily. |
| David Brayley submitted a diabetic diagnosis for Roberta Fuentes, 123444 with instructions for Colin Hung to provide insulin two times daily. |
| |
| Colin Hung checked the blood sugar levels of four (4) diabetic patients and, based upon the results, prepared insulin injections. During the process Colin Hung called for Bonnie Smith to step forward and receive care. Roberta Fuentes appeared and presented herself for an injection of insulin. The nurse failed to check the patient's wristband. Upon discovering his mistake, the nurse provided Bonnie Smith with the intended |
| insulin injection and monitored Roberta Fuentes who received the medication in error. Neither patient required additional treatment. |

The redacted document may include the following redacted memo, where the sensitive data is replaced with linked descriptor labels:

| Incident Summary: [Patient], [Patient No.] |
|---|
| [Doctor] submitted a diabetic diagnosis for [Patient], [Patient No.] with instructions for [Nurse] to provide insulin three times daily. |
| [Doctor] submitted a diabetic diagnosis for [Patient], [Patient No.] with instructions for [Nurse] to provide insulin two times daily. |
| |
| [Nurse] checked the blood sugar levels of four (4) diabetic patients and, based upon the results, prepared insulin injections. During the process [Nurse] called for [Patient] to step forward and receive care. [Patient] appeared and presented herself for an injection of insulin. The nurse failed to check the patient's wristband. Upon discovering his mistake, the nurse provided [Patient] with the intended insulin injection and monitored [Patient] who received the medication in error. Neither patient required additional treatment. |

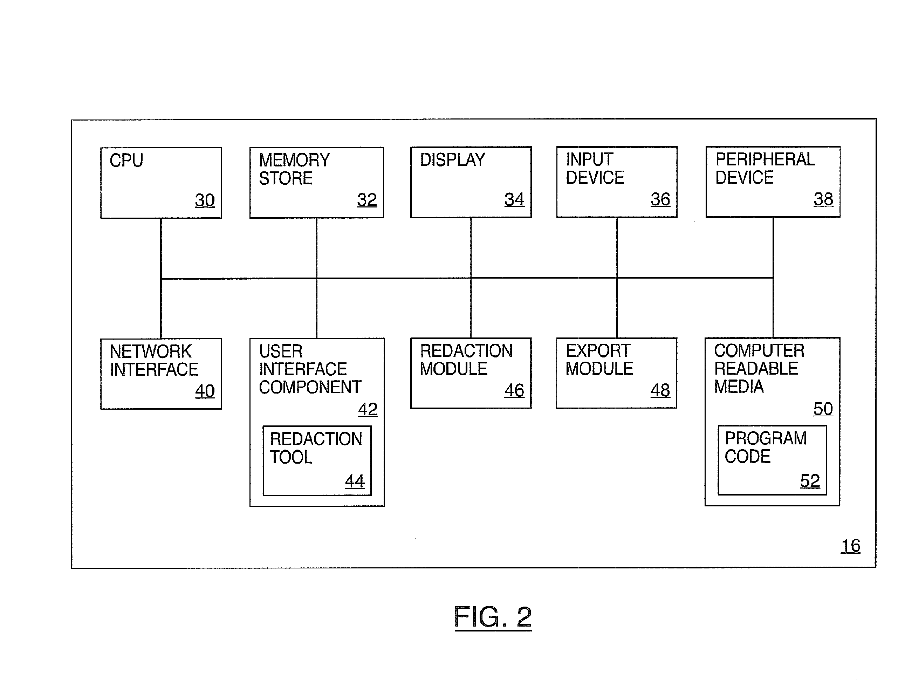

Figure 2 is a block diagram illustrating the components of a workstation 16 of a system 10 for redacting sensitive data entries in accordance with an example embodiment.

Workstation 16 may be any networked computing device including a processor and memory, such as a personal computer, workstation, server, portable computer, mobile phone, personal digital assistant, laptop, smart phone, satellite phone, WAP phone, or a combination of these. Workstation 16 may include a software application, application plug-in (e.g. a widget), instant messaging application, mobile device application, e-mail application, online telephony application, java application, web page, or web object (e.g. a widget) residing or rendered on workstation 16 in order to access data system 12 using network 16.

In an exemplary embodiment, workstation 16 includes a central processing unit 30, a memory store 32, a display 34, an input device 36, one or more peripheral devices 38, a network interface 40, a user interface component 42, an export module 48 and a computer readable media 50. Workstation may also include a redaction module 46, or alternatively may access the redaction module 24 of data system 12 via network. The functionality of redaction module 46 is the same as described in relation to redaction module 24.

The display 34 is a monitor type device that is used to display information. The input devices 36 may be any device that allows for input, examples of which may include, but are not limited to, keyboards, touch screens, microphones, speakers, and pointing devices. The memory store 32 is a permanent storage associated with the workstation 16. The central proccessing unit 42 is used to execute instructions or program code 52 stored on computer readable media 50 or memory store 32. The program code 52 on computer readable media 50 may also be stored on memory store 32. The network interface 40 may be a wired and/or wireless network interface that allows the device to connect to the network 14. The peripheral devices 38 may include but are not limited to, devices such as printers, antenna, transceivers and scanners.

User interface component 42 may include program code defining how an application outputs information to a user during execution of an application, and can be implemented as command driven, menu driven, and graphical interface driven, for example. User interface component 42 is operable to provide a document with sensitive data entries on display 34 for review by a user of the workstation 16. User interface component 42 is further operable to provide a toolbar component with selectable tools on display 14, including a redaction tool 44 to initiate a command to redact one or more sensitive entries.

The user interface component 42 receives a command to redact one or more sensitive entries via input device 36. The command to redact may be received by the redaction tool 44, or when a user highlights or drags over a data entry using input device 36. In addition, the command to redact may be received automatically when user requests a document for export.

The redaction module 46 generates a redacted document and the user interface component 42 may provide the redacted document on display 42 for preview by the user. In addition, the user interface module 42 may receive a command to export the document and may provide the redacted document to export module 48. The export module 48 and peripheral devices 38 are operable to export the redacted document by printing, file transmitting, saving, and email, for example. Further, data system 12 is operable to encrypt the redacted document for export via file transmission, email, and the like.

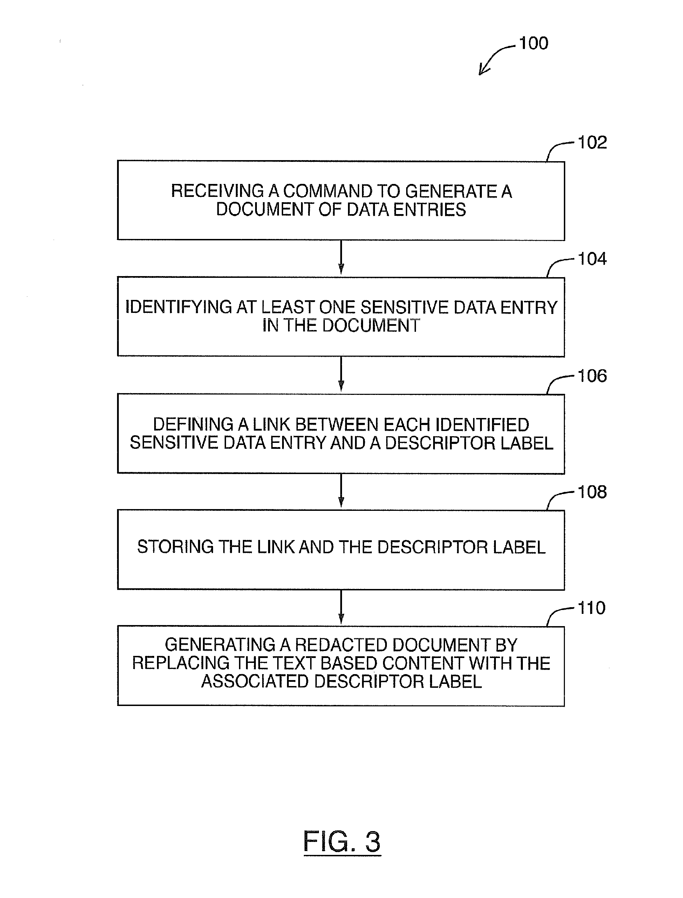

Figure 3 is a flow diagram of a method 100 for redacting sensitive data entries in accordance with an example embodiment.

The process begins at step (102), where health care incident management system 12 receives a command to generate a document of data entries from user interface component 42 of workstation 16. The health care incident management system 12 generates the document using text-based content associated with the data entries. The document may be stored on workstation 16, or a networked persistent store (remote or local) accessible to user interface component 42.

The document may be a form, a field, a report, a memo, an email, a fax, and may include text, tables, figures, pictures, attachments, abstracts, summaries, appendices, footnotes, hyperlinks, charts, graphs and the like.

Sensitive information may include personal, private, privileged, classified, secret, and confidential information that is not suitable for distribution to one or more recipients. For example, sensitive information may be private health information, such as a patient's name, address, phone number, MRN, doctor's name, nurse's name, room number, name of patient's relatives or representatives.

The form engine 20 generates a form using a form template. The form includes form fields configured to receive form field data values. The form fields are instances of field objects, which define a set of attributes for form fields. If health care incident management system 12 receives form field data values then health care incident management system 12 is operable to create an incident file record and store the form field data values in database 26 in association with the incident file record. Health care incident management system 12 may associate the form field data value with a data type, caption, and other attributes defined by the field object used to generate the form field. Form fields may include web forms, memo fields, text fields, radio button fields, drop down fields, checkbox fields, pick tree fields, file selects, buttons, and the like.

The report engine 22 generates reports using data values (including form field data values) stored in database 26. The report engine 22 is operable to configure a report summary rendering engine to generate a report summary document in a mark up language such as html, for example. The html document may be converted into a redacted document in a format suitable for export such as pdf. The html document may be stored in database 26 or may be stored temporarily in memory.

At step (104), redaction module 24/46 identifies at least one sensitive data entry in the document. The redaction module 24/46 is operable to identify the sensitive data entries in response to receiving a command to redact one, some or all of the sensitive data entries. The redaction module 24/46 is further operable to identify a sensitive data entry in response to receiving selected text from user interface component 42 or upon determining that a data entry is of a sensitive data type.

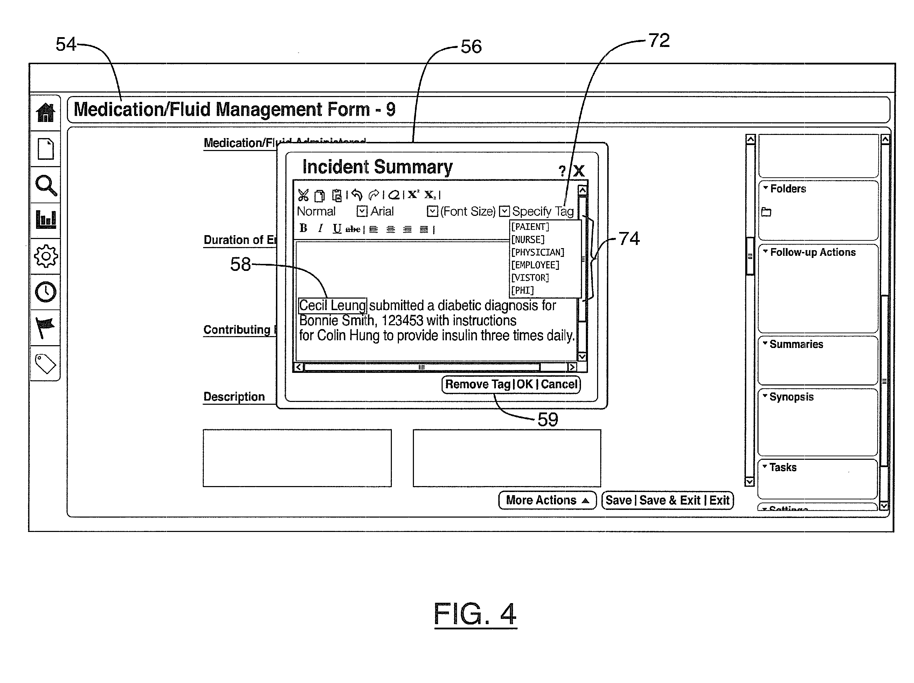

Figures 4, 6, 7, and 8 illustrate example user interface component 42 embodiments. Figure 4 illustrates that user interface component 42 is operable to display sensitive data entry 58 as being selectable for redaction. The user interface component 42 implements a redaction tool 44 to select text for redaction. When a user selects text using input device 36 of workstation 16, then user interface component 42 is operable to receive the command to redact and relays the command to redaction module 24/46. The sensitive data entry 58 can be a single word, a group of words, a part of the word, a section of the document, pages of the document, the entire document, an image, text or a file attachment, for example. The user interface component 42 is further operable to highlight or otherwise identify the selected sensitive data entry 58. In this example, the user interface component 42 is further operable to implement an unredaction tool 59 to deselect or unredact a previously selected or redacted sensitive data entry 58. When a sensitive data entry 58 is selected or redacted and user interface component 42 receives a command from the unredaction tool 59 to deselect or unredact the sensitive data entry 58, then the data entry 58 is no longer identified as sensitive and is no longer redacted. The data system 12 is further configured to modify the descriptor label associated with a specific sensitive data field or no longer associate the descriptor label with a specific data field.

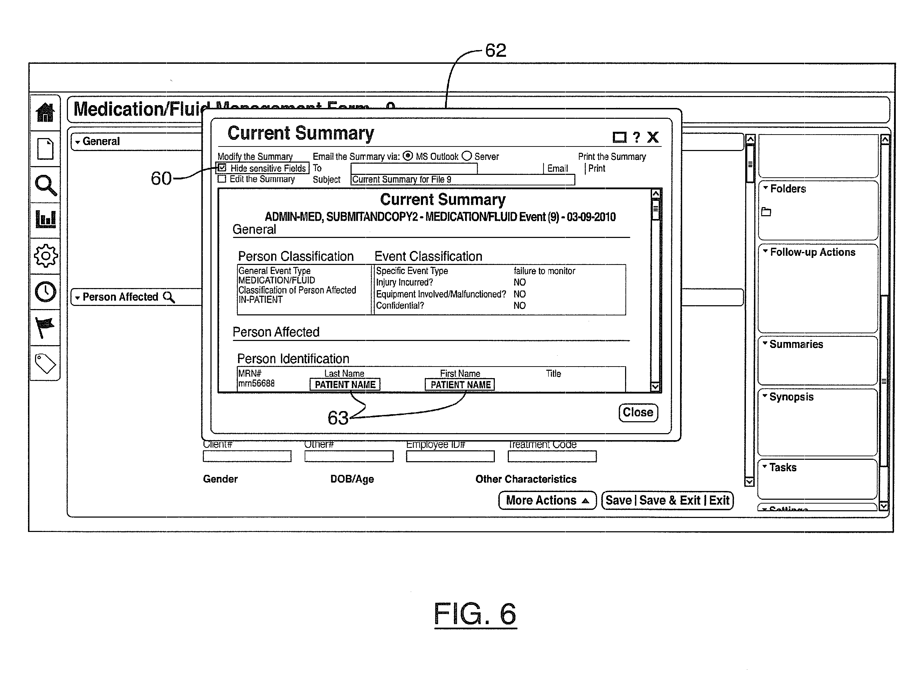

Figure 6 illustrates that user interface component 42 is operable to display a form 62 and receive the command to redact via a form checkbox 60, or other form component. The user interface component 42 is operable to display sensitive data entries 63 within a form. As shown, the sensitive data entries 63 may be form field values stored in database 26. In this example, the user interface component 42 receives a command to redact all sensitive data entries in the document when the form checkbox 60 is selected. In response, redaction module 24/46 is operable to redact form field values that are sensitive data entries 63 and the user interface component 42 is operable to display the redacted sensitive data entries 63 in the form.

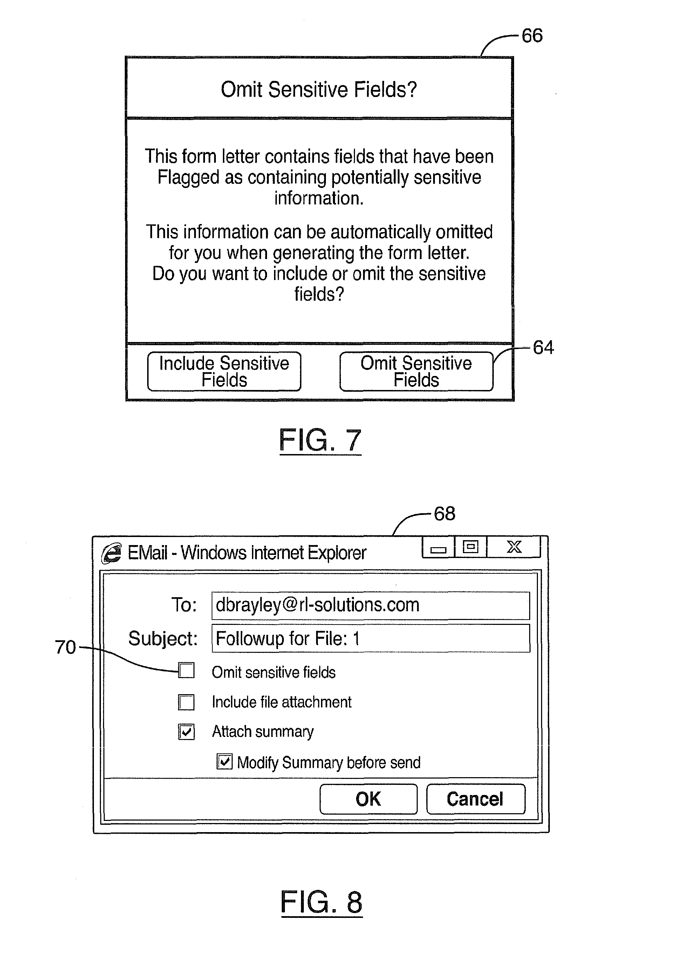

Figure 7 illustrates that user interface component 42 is operable to provide a popup dialogue box 66 and a selectable indicia 64 to receive the command to redact. Health care incident management system 12 is operable to determine whether the document includes sensitive data entries and if so prompts the user to command that none, some or all of the sensitive data entries should be redacted.

Figure 8 illustrates that that user interface component 42 is operable to provide a dialogue box 68 for to receive a command to export the document, including a selectable indicia 70 to initiate a command to redact. This example illustrates that redaction module 24/46 can receive a command to redact upon receiving a command to generate a document for export.

These are merely illustrative examples, and user interface component 42 may provide other mechanisms for receiving a command to redact such as via menu options, toolbar components, mouse events, keyboard events, and the like.

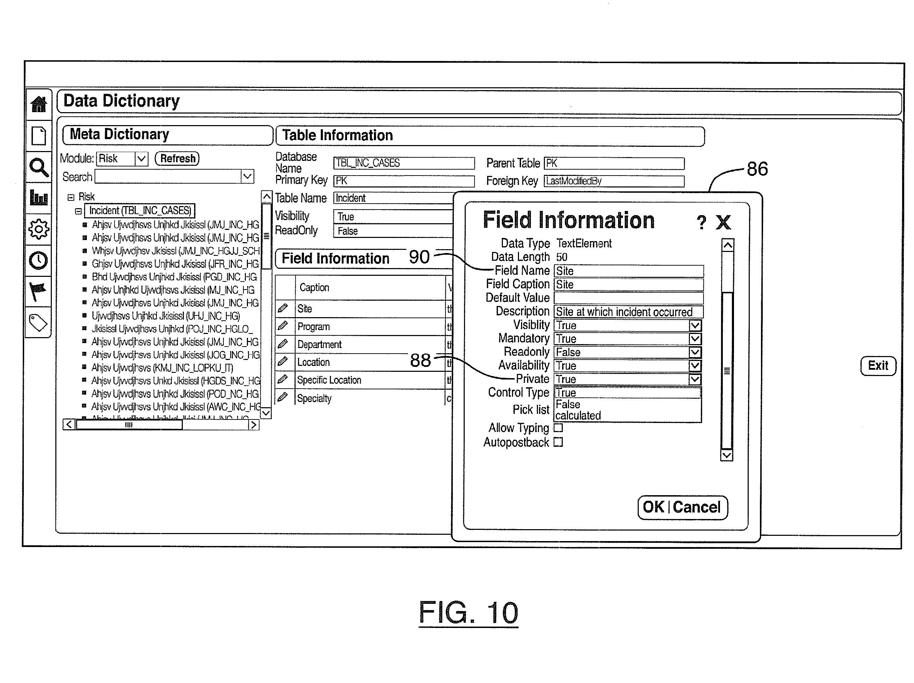

At step (106), redaction module 24/46 defines a link between each identified sensitive data entry and a descriptor label in database and, at step (108), stores the link and the descriptor label in a repository, such as database 26. For example, as shown in Figure 11, the sensitive data entry 92 may be the doctor's name and the descriptor label 96 may be "DOCTOR". As another example, as shown in Figure 10, the sensitive data entry may be the site where a patient incident occurred and the descriptor label 90 may be "SITE". As a further example, if the entire document or an attachment to the document is sensitive then the text or attachment file name may be replaced with "confidential data", "patient personal data", and the like.

At step (110) redaction module 24/46 generates a redacted document by, for each identified sensitive data entry, replacing the text-based content with the associated descriptor label. To generate the redacted document, the redaction module 24/46 replaces the text in a copy of the document with the descriptor label text to generate the redacted document. For example, a 10 character string will be replaced with a 5 character string.

For example, the document may include the following memo:

| Incident Summary: Roberta Fuentes, 123444 |
|---|
| Cecil Leung submitted a diabetic diagnosis for Bonnie Smith, 123453 with instructions for Colin Hung to provide insulin three times daily. |
| David Brayley submitted a diabetic diagnosis for Roberta Fuentes, 123444 with instructions for Colin Hung to provide insulin two times daily. |
| |
| Colin Hung checked the blood sugar levels of four (4) diabetic patients and, based upon the results, prepared insulin injections. During the process Colin Hung called for Bonnie Smith to step forward and receive care. Roberta Fuentes appeared and presented herself for an injection of insulin. The nurse failed to check the patient's wristband. Upon discovering his mistake, the nurse provided Bonnie Smith with the intended insulin injection and monitored Roberta Fuentes who received the medication in error. Neither patient required additional treatment. |

The redacted document may include the following redacted memo:

| Incident Summary: [Patient], [Patient No.] |
|---|
| [Doctor] submitted a diabetic diagnosis for [Patient], [Patient No.] with instructions for [Nurse] to provide insulin three times daily. |

| [Doctor] submitted a diabetic diagnosis for [Patient], [Patient No.] with instructions for [Nurse] to provide insulin two times daily. |
|---|
| |
| [Nurse] checked the blood sugar levels of four (4) diabetic patients and, based upon the results, prepared insulin injections. During the process |
| [Nurse] called for [Patient] to step forward and receive care. [Patient] appeared and presented herself for an injection of insulin. The nurse failed to check the patient's wristband. Upon discovering his mistake, the nurse provided [Patient] with the intended insulin injection and monitored [Patient] who received the medication in error. Neither patient required additional treatment. |

In this example, the document includes a memo generated using form field value data received via a form (not shown) at workstation 16. As shown in the above example, all highlighted sensitive entries in the document are replaced with descriptor labels in the redacted document. The descriptor labels may help a recipient of the redacted document better understand that content of the redacted document, while still anonymizing the sensitive information.

The memo shown may be generated using form fields, such as the form field "patient name", "nurse name" and "doctor name". These form fields may be instances of field objects that define a set of attributes for the form fields, such as name, caption, description, etc. For example, an attribute for a form field may be 'caption', where the caption for the form field "patient name" is "patient". The user interface module 42 may display caption adjacent the form field in the form. This caption may be used as a descriptor label for the form field value. In other embodiments, any attribute for the form field may be used as the descriptor label for a corresponding sensitive data entry that was received at a form field in a form.

The user interface module 42 may display the redacted document on a display 34 for a user to review. After viewing the displayed redacted document, the user interface module 42 may receive a command to further redact the document if a sensitive data entry was not selected initially, for example. The user interface module 42 is further operable to receive a command to unredact one, some, or all of sensitive data entries in the redacted document.

The user interface module 42 is further operable to receive a command to modify a descriptor label. In this example, a descriptor label for the sensitive data entry "patient name" is "patient" and there are two different field values for "patient name" shown in this example, namely, "Bonnie Smith" and "Roberta Fuentes". Redaction module 24/46 is operable to determine that two different sensitive data entries have the same linked descriptor labels. The user interface module 42 may prompt for a command to modify the descriptor label for 'patient name' or otherwise receive a command to modify the descriptor label for 'patient name'. The user interface module 42 may be operable to automatically modify the descriptor labels. In this example, the descriptor labels "patient" may be modified to 'patient A' and 'patient R' in order to distinguish between the different two field values. Redaction module 24/26 is operable to modify the linked descriptor labels and generates a new redacted document.

The new redacted document may include the following redacted memo:

| Incident Summary: [Patient B], [Patient No.] |
|---|
| [Doctor] submitted a diabetic diagnosis for [Patient A], [Patient No.] with instructions for [Nurse] to provide insulin three times daily. |
| [Doctor] submitted a diabetic diagnosis for [Patient B], [Patient No.] with instructions for [Nurse] to provide insulin two times daily. |
| |
| [Nurse] checked the blood sugar levels of four (4) diabetic patients and, based upon the results, prepared insulin injections. During the process [Nurse] called for [Patient A] to step forward and receive care. [Patient B] appeared and presented herself for an injection of insulin. The nurse failed to check the patient's wristband. Upon discovering his mistake, the nurse provided [Patient A] with the intended insulin injection and monitored [Patient B] who received the medication in error. Neither patient required additional treatment. |

This example illustrates that the descriptor labels may be predetermined descriptor labels associated with a field value such as "patient", "nurse", and "doctor". The descriptor labels may also be user-defined descriptor labels.

In some embodiments, redaction module 24/46 may receive a single command to redact all sensitive data entries in the document. In response, redaction module 24/46 identifies all sensitive data entries in the document and replaces all the sensitive text-based content with their associated descriptor label to generate the redacted document.

In some embodiments, the user interface module 42 may receive a command to export the document, such as via print, save, file transmission and email. The command to export may contain a recipient identifier that identifies a recipient of the document being exported. Redaction module 24/46 may define a set of sensitive data entries linked to the recipient identifier. This feature allows redaction module 24/46 to identify and redact different sets of sensitive data entries depending on the recipient of the document. For example, a recipient internal to the organization may have a smaller set of sensitive data entries then a recipient external to the organization, for example.

In further embodiments, redaction module 24/46 receives a command to generate the document comprising a user identifier. Redaction module 24/46 is operable to identify one or more sensitive data entries based on the user identifier. The user may customize a set of sensitive data entries to be redacted from their documents, and link the set of sensitive data entries to their user identifier. Redaction module 24/26 may retrieve the set from database 26 using the user identifier and identify sensitive data entries using the set of configured sensitive data entries. For example, a first user may configure a set of sensitive data entries to include data entries having the data type "name", and redaction module 24/26 links that set of sensitive data entries to a first user identifier. A second user may configure a set of sensitive data entries to include data entries having the data type "name", "home address", and "home phone number", and redaction module 24/26 links that set of sensitive data entries to a second user identifier. If a document includes data entries of data type name, home address and home phone number then redaction module 24/26 is operable to generate a different redacted document for the first and second user based on their user identifiers.

In further embodiments, user interface component 42 may provide a set of descriptor labels 74, where each descriptor label is selectable. When user interface component 42 receives a selected descriptor label, user interface component 42 provides the selected descriptor label to the redaction module 24/46. Redaction module 24/46 defines a link between the selected sensitive data entry and the received descriptor label, and stores the link in the database 26.

Figure 4 illustrates the user interface component 42 displaying text 58 as being selectable for redaction. The user interface component 42 implements a redaction tool 44 to select a sensitive data entry for redaction. In this example, when user interface component 42 receives selected sensitive data from, for example, input device 36 of workstation 16, then the user interface component 42 receives the command to redact the selected sensitive data entry. In response, user interface component 42 is operable to provide a set of descriptor labels 74, where each descriptor label is selectable. This provides user with a range of selectable options of descriptor labels to replace the sensitive text in the redacted document. Using a descriptor label to replace the sensitive text may make the redacted document more understandable to the recipient. User interface component 42 is further operable to modify the set of descriptor labels 74 depending on the data type of the selected sensitive data entry. For example, user interface component 42 is operable to determine that the selected sensitive data entry is of data type "name" and provide a set of descriptor labels 74 associated with a name, such as patient, nurse, physician, employee, and visitor, for example.

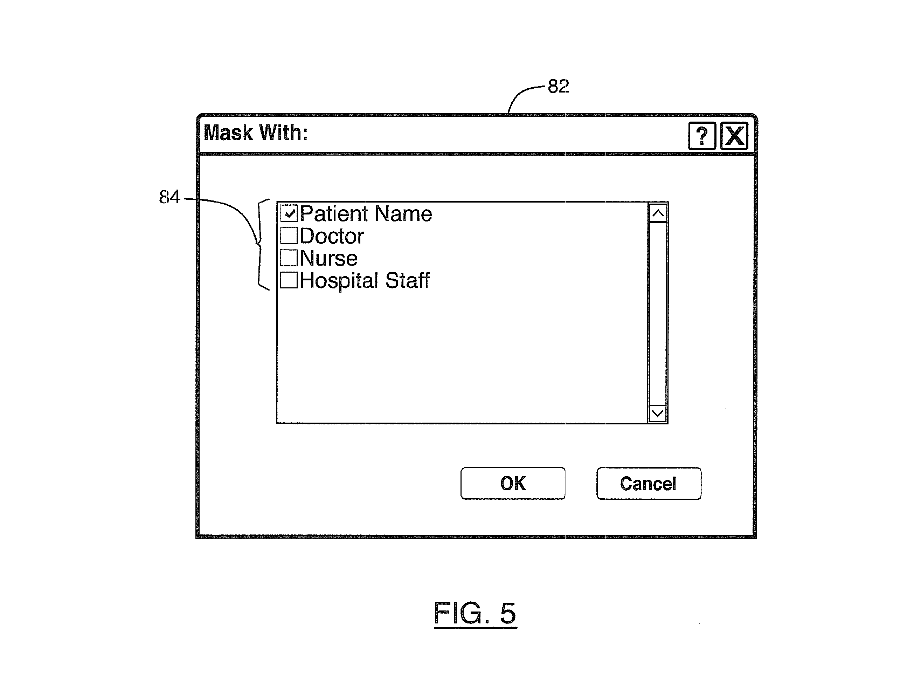

As another example, Figure 5 illustrates user interface component 42 providing a set of selectable descriptor labels 84 in a dialogue box 82. User interface component 42 provides the set to receive at least one selected descriptor label for an identified sensitive data entry. For example, user interface component 42 provides a popup dialogue box to prompt the user to select a descriptor label to replace the selected text from: patient name, doctor, nurse, and hospital staff.

The request to redact one or more sensitive data entries may include a user identifier. Redaction module 24/46 may link a set of selectable descriptor labels 84 to a specific user identifier, so that the set of selectable descriptor labels 84 provided by user interface component 42 is custom to the user identifier and may vary depending on the specific user.

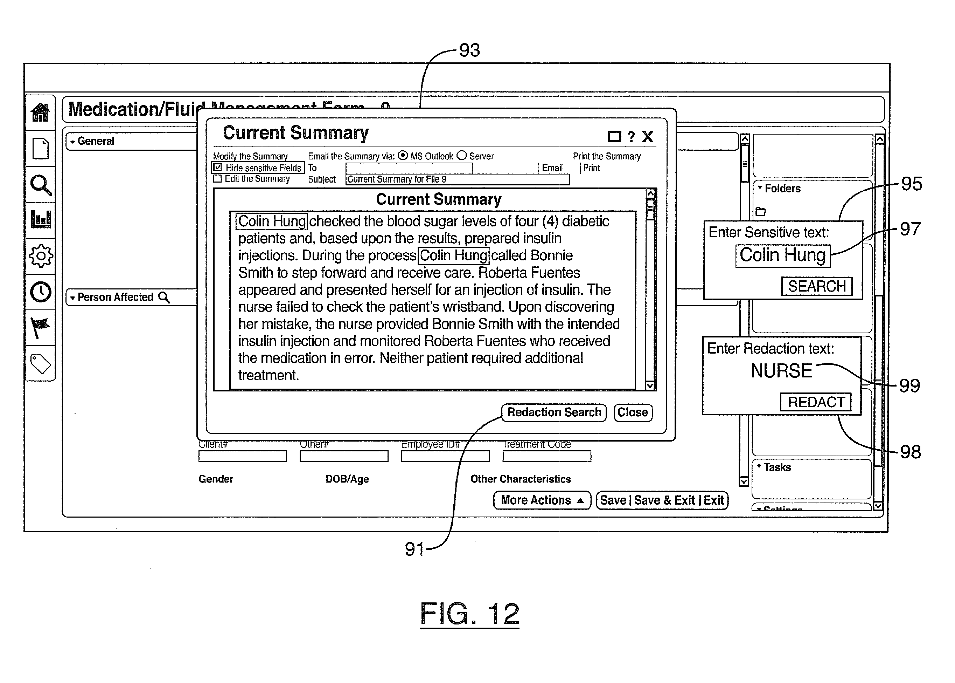

In some embodiments, the user interface component 42 may receive sensitive text-based content at a text box. Figure 12 illustrates that the user interface component 42 is operable to implement a redaction search tool 91 to receive sensitive text-based content. Redaction module 24/26 is operable search the text in a document for the sensitive text-based content to identify sensitive data entries. If the redaction module 24/26 determines that a data entry in the document comprises the sensitive text-based content, then data system 12 marks that data entry as a sensitive data entry. User interface component 42 may highlight all identified sensitive data entries in the document.

The user interface component 42 is operable to customize the description of the sensitive data to be used in the redacted document. For example, user interface component 42 may provide a dialogue box 98 with a text field 99 to receive a custom descriptor label for all sensitive data entries that the redaction module 24/26 identified in the search, or otherwise.

In further embodiments, data system 12 determines whether the document comprises one or more sensitive data entries by associating at least one data entry in the document with a data type. For example, the data entry "Bonnie Smith" may be associated with the data type "patient name". The redaction module 24/46 is operable to define at least one data type as a sensitive data type. In this example, redaction module may define the data type "patient name" as a sensitive data type. The redaction module 24/46 searches document text for data entries associated with a sensitive data type. In this example, redaction module 24/46 searches for data entries of data type "patient name" in the document, such as "Bonnie Smith". When redaction module 24/46 identifies a data entry of a sensitive data type then redaction module 24/46 identifies the data entry as a sensitive data entry. In this example, redaction module 24/46 identifies the data entry "Bonnie Smith" in the document as a sensitive data entry.

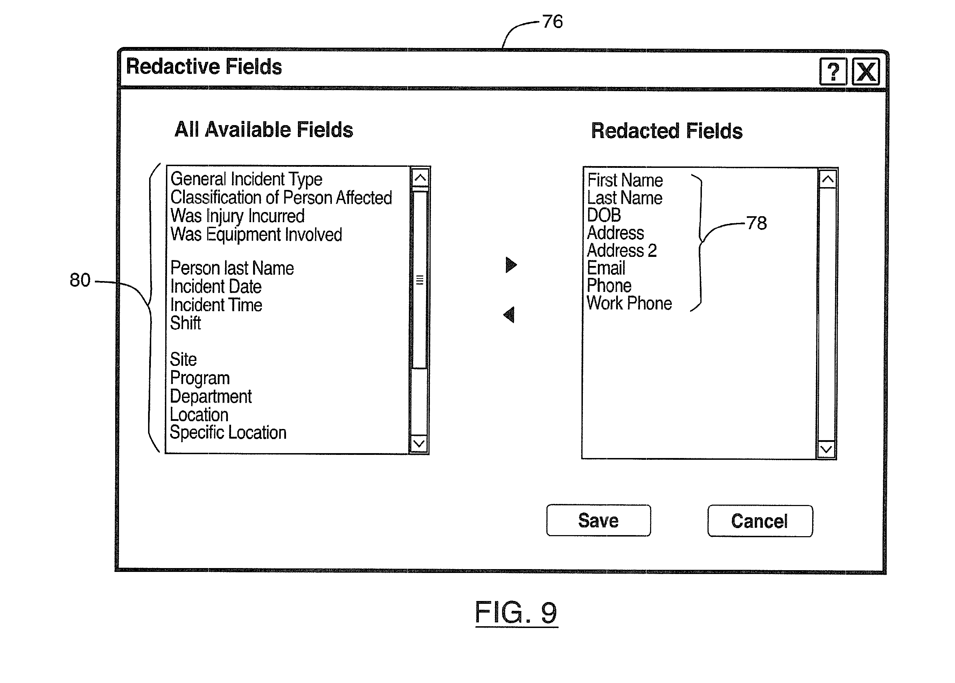

Figure 9 illustrates a graphical user interface component 76 for marking data types as sensitive. A user can configure a set of sensitive data types 78 by selecting data types from a displayed set of all data types in the document 80. When a sensitive data type is added to the set of sensitive data types 78, redaction module 24/46 defines a link between each sensitive data type and an associated descriptor label.

In further embodiments, when one data entry is identified as a sensitive data entry then redaction module 24/46 may search document for the sensitive text associated with the identified sensitive data entry to identify additional sensitive data entries. This provides an efficient mechanism to identify sensitive data entries as a user of the system 10 does not have to manually identify each individual instance of the sensitive text based content after initially identifying the sensitive text the first time.

As shown in these illustrative examples, the descriptor labels may be predetermined descriptor labels, such as the set of descriptor labels 84 shown in Figure 5. The descriptor labels may also be a user-defined or a custom descriptor label, such as received via a text box 99, for example.

As indicated herein, the data entries in the document may be received at user interface component 42 via a form, which is a collection of form fields.

Form engine 20 receives form field values and stores the form field values in the database 26. Report engine 22 may access database 26 to generate a document using data entries that are form field values.

Form engine 24/46 defines a link between the form field data value and a corresponding form field object. A form field is an instance of a form object and is configured to receive the form field value. The form engine defines attributes for form field objects, which in turn define attributes for form fields. The form object attributes may include a sensitive data determination attribute.

Figure 10 illustrates an example user interface component 86 with a sensitive data determination attribute 88 for a form field object. Redaction module 24/26 determines that a data entry in the document is a sensitive data entry using the sensitive data determination attribute of its corresponding form field object. The form object attributes include a caption attribute, which may be displayed in association with the form field in a form. For example, the form may be an incident tracking form used on workstations 16 in a health care facility. The field object may define attributes for the site where a care incident occurred, and the caption attribute 90 may comprise the text "site". The redaction module 24/26 may define the linked descriptor label as the caption attribute of the corresponding form field object.

An administrative user may access user interface component 86 via workstation 16 to set the sensitive data determination attribute 88 of form field objects. By marking the sensitive data determination attribute 88 as true, all data entries corresponding to the field object will be replaced with the linked descriptor label text, such as the caption attribute 90, in the redacted document. This feature enables all sensitive data types to be automatically redacted without requiring individual manual selection.

To change the descriptor label text an administrative user can change the parameter of the attribute for the descriptor label text via a user interface component 86.

In some embodiments, the document and redacted document are generated in a mark up language, such as html. For example, referring to Figure 6 the document may be a form 62 and the user interface component 42 is operable to display sensitive data entries 63 within the form 62. In this example, the user interface component 42 is operable to receive a command to redact all sensitive data entries in the document when the form checkbox 60 is selected.

As described above, redaction module 24/46 is operable to identify sensitive data entries in the form 62. For example, redaction module 24/26 is operable to determine that a field value displayed in the form 62 is a sensitive data entry using the sensitive data determination attribute of the corresponding form field object. When a data entry in the form 62 is identified as a sensitive data entry then the redaction module 24/46 is operable to generate a mark up language attribute tag in the html document indicating that the field value is a sensitive data entry 63.

For example, a portion of the document and redacted document may be generated in the mark up language html using the following source code:

```
 <div class="clusterRow"><table class="DataFormField"
 id="ctl02_FallMgmt_INCPERSON_ctl05 ctl08" fId="197"
 style="width:19.9%;"><tr><td><align="left"><span
 class="editableElem">MRN#</span></td></tr><tr><td class="DataFormFieldEditor"
 id="id_197"><span class="editableElem
 F197">mrn56688</span></td></tr></table><table class="DataFormField"
 id="ctl02_FallMgmt_INCPERSON_ctl05_ctl09" fId="178"
 style="width:29.999%;"><tr><td align="left"><span class="editableElem">Last
 Name</span></td></tr><tr><td class="DataFormFieldEditor" id="id_178"><span
 class="editableElem F178 Redact">JACK</span></td></tr></table><table
 class="DataFormField" id="ct102_FallMgmt_INCPERSON_ct105_ct110" fId="180"
 style="width:29.999%;"><tr><td align="left"><span class="editableElem">First
 Name</span></td></tr><tr><td class="DataFormFieldEditor" id="id_180"><span
 class="editableElem F180 Redact">DAWSON</span></td></tr></table><table
 class="DataFormField" id="ct102_FallMgmt_INCPERSON_ct105_ct111" fId="177"
 style="width:18.9%;"><tr><td align="left"><span
 class="editableElem">Title</span></td></tr><tr><td class="DataFormFieldEditor"
 id="id_177"><span class="editableElem F177
 "> </span></td></tr></table></div>
                                                       </div></td>
```

Health care incident management system 12 generates the document as html with the form fields LAST NAME and FIRST NAME associated with the mark up language attribute tag "Redact". In this example, the form fields LAST NAME and FIRST NAME are initially identified as sensitive data entries 63 based on the sensitive data determination attribute of the corresponding form field object (e.g. the value is set to TRUE).

In response to the form checkbox 60 being selected, redaction module 24/46 generates the redacted document by replacing the sensitive data entries 63 with the linked descriptor labels. The mark up language attribute tag instructs redaction module 24/46 when generating a redacted document, at print or export runtime for example, that the sensitive field should be replaced with linked descriptor labels. In this example, the mark up language attribute tag instructs the redaction module 24/46 to replace LAST NAME and FIRST NAME with linked descriptor labels. If the form checkbox 60 is not selected then the document is generated using the form field values and not the descriptor label.

The data system 12 is further configured to unmark a sensitive data entry If the data entry is no longer sensitive, then the mark up language attribute tag is removed from the html document. For example, if the form field values LAST NAME and FIRST NAME are subsequently unmarked using the sensitive data determination attribute (e.g. the value is set to FALSE) then health care incident management system 12 generates the document as html without associating the mark up language attribute tag "Redact" with those form fields. In this case, when the redaction module 24/46 receives a command to redact then the redaction module will not replace those field values with the descriptor labels when generating a redacted document.

Numerous specific details are set forth herein in order to provide a thorough understanding of the exemplary embodiments described herein. However, it will be understood by those of ordinary skill in the art that these embodiments may be practiced without these specific details. In other instances, well-known methods, procedures and components have not been described in detail so as not to obscure the description of the embodiments. Furthermore, this description is not to be considered as limiting the scope of these embodiments in any way, but rather as merely describing the implementation of these various embodiments.

Various aspects and features of the present invention are set out in the following numbered clauses:
1. A method for redacting sensitive data entries, wherein the method is implemented on a processor having access to a memory in which instructions are stored, the instructions being executable to configure the processor to perform operations comprising:
   receiving a command to generate a document, wherein the document comprises data entries, wherein each data entry comprises text-based content;
   identifying at least one sensitive data entry in the document;
   defining a link between each identified sensitive data entry and a descriptor label;
   storing the link and the descriptor label in a repository; and
   generating a redacted document by, for each identified sensitive data entry,
   replacing the text-based content with the associated descriptor label.
2. The method of clause 1 further comprising:
   receiving a command to redact at least one sensitive data entry in the document; and
   generating the redacted document by, for each of the at least one sensitive data entries in the command to redact, replacing the text-based content with the associated descriptor label.
3. The method clause 1 further comprising:
   receiving a single command to redact all sensitive data entries in the document;
   identifying all sensitive data entries in the document; and
   generating the redacted document by, for each sensitive data entry in the document, replacing the text-based content with the associated descriptor label.
4. The method of clause 1 wherein the command to generate the document comprises a user identifier, and wherein at least one identified sensitive data entry in the document is identified based on the user identifier.
5. The method of clause 1 further comprising receiving a command to export the document; and exporting the redacted document.
6. The method of clause 5 therein the command to export comprises a recipient identifier, and wherein at least one identified sensitive data entry in the document is identified based on the recipient identifier
7. The method of clause 5 wherein exporting is selected from the group consisting of: printing, saving, transmitting and emailing.
8. The method of clause 1 wherein each sensitive data entry is selectable, and wherein the method further comprises:
   receiving a selected sensitive data entry;
   receiving a descriptor label; and
   defining a link between the selected sensitive data entry and the received descriptor label.
9. The method of clause 1 wherein each sensitive data entry is selectable, and wherein the method further comprises:
   receiving a selected sensitive data entry;
   providing a set of descriptor labels, wherein each descriptor label is selectable;
   receiving a selected descriptor label; and
   defining a link between the selected sensitive data entry and the selected descriptor label.
10. The method of clause 1 wherein determining whether the document contains one or more sensitive data entries comprises:
   receiving sensitive text-based content;
   determining whether a data entry in the document comprises the sensitive text-based content; and
   upon determining that the data entry in the document comprises the sensitive text-based content, determining that the data entry is a sensitive data entry.
11. The method of clause 1 wherein determining whether the document comprises one or more sensitive data entries comprises:
   associating at least one data entry in the document with a data type;
   defining at least one data type as a sensitive data type;
   determining, for each of the at least one data entry in the document, whether the associated data type is a sensitive data type; and
   upon determining that the associated data type is a sensitive data type, determining that the data entry is a sensitive data entry.
12. The method of clause 1 wherein the identified sensitive data entry comprises sensitive text-based content; wherein at least one additional data entry in the document comprises the sensitive text-based content; and wherein the method further comprises:
   identifying the at least one additional data entry as a sensitive data entry using the sensitive text-based content;
13. The method of clause 1 wherein at least one descriptor label is selected from a set of predetermined descriptor labels.
14. The method of clause 1 wherein at least one descriptor label is a user-defined descriptor label; wherein the method further comprises receiving the user-defined descriptor label.
15. The method of clause 1 wherein defining a link between each sensitive data entry and a descriptor label further comprises:
   defining, for each sensitive data entry, a link between the sensitive data entry and a data type; and
   defining a link between each data type and a descriptor label.
16. The method of clause 1 wherein the at least one data entry in the document comprises a form field data value; and wherein the method further comprises:
   defining a link between the form field data value and a corresponding form field object, wherein the field object is configured to define a form field, wherein the form field is configured to receive the form field data value;
   defining attributes for the form field object, wherein the attributes comprise a sensitive data determination attribute and a caption attribute;
   determining that the at least one data entry in the document is a sensitive data entry based on the sensitive data determination attribute of the corresponding form field object; and
   defining the linked descriptor label using the caption attribute of the corresponding form field object.
17. The method of clause 1 further comprising:
   providing the document using a mark up language, wherein the document comprises a mark up language attribute tag for each sensitive data entry in the document;
   identifying a sensitive data entry using the mark up language attribute tag in the document; and
   generating the redacted document using the mark up language attribute tag for the sensitive data entry.
18. The method of clause 1 wherein the document selected from the group consisting of a picture, a form, a field, a report, a memo, and attachment to a form.
19. The method of clause 17 wherein the descriptor label is any html enabled object.
20. A method for redacting sensitive data entries, the method comprising:
   receiving a command to generate a document, wherein the document comprises data entries, wherein each data entry comprises text-based content;
   receiving a command to export the document;
   receiving a single command to redact all sensitive data entries in the document;
   identifying all sensitive data entries in the document;
   generating a redacted document by, for each sensitive data entry in the document, replacing the text-based content with the associated descriptor label; and
   exporting the redacted document.
21. A computing system for redacting sensitive data entries comprising:
   at least one processor and at least one memory, wherein the processor is configured to execute instructions stored in the memory to provide:
      a user interface component configured to:
         receive a command to generate a document wherein the document comprises data entries, wherein each data entry comprises text-based content;
         provide a redacted document;
      redaction module configured to:
         identify at least one sensitive data entry in the document;
         define a link between each identified sensitive data entry and a descriptor label;
         store the link and the descriptor label in a repository; and
         generate a redacted document by, for each identified sensitive data entry, replacing the text-based content with the associated descriptor label.
22. The system of clause 21 wherein the user interface component is further configured to receive a command to redact at least one sensitive data entry in the document; and wherein the redaction module is further configured to generate the redacted document by, for each of the at least one sensitive data entries in the command to redact, replacing the text-based content with the associated descriptor label.
23. The system of clause 21 wherein the user interface component is further configured to receive a single command to redact all sensitive data entries in the document; and wherein the redaction module is further configured to identify all sensitive data entries in the document, and to generate the redacted document by, for each of the at least one sensitive data entries in the document, replacing the text-based content with the associated descriptor label.
24. The system of clause 21 wherein the command to generate the document comprises a user identifier; and wherein the redaction module is further configured to identify at least one sensitive data entry in the document based on the user identifier.
25. The system of clause 21 wherein the user interface component is further configured to receive a command to export the document; and the system further comprises an export module configured to export the redacted document.
26. The system of clause 25 wherein the command to export comprises a recipient identifier, and wherein the redaction module is further configure to identify at least one sensitive data entry in the document based on the recipient identifier.
27. The system of clause 25 wherein the export module is configured to export the redacted document from the group consisting of: print, save, transmit and email.
28. The system of clause 21 wherein the user interface component is configured to provide each sensitive data entry as selectable, and wherein the redaction component is further configured to:
   receive a selected sensitive data entry;
   receive a descriptor label; and
   define a link between the selected sensitive data entry and the received descriptor label.
29. The system of clause 21 wherein the user interface component is configured to provide each sensitive data entry as selectable and wherein the redaction component is further configured to:
   receive a selected sensitive data entry;
   provide a set of descriptor labels, wherein each descriptor label is selectable;
   receive a selected descriptor label; and
   define a link between the selected sensitive data entry and the selected descriptor label.
30. The system of clause 21 wherein the redaction module is configured to:
   associate at least one data entry in the document with a data type;
   define at least one data type as a sensitive data type;
   determine that at least one data entry in the document is associated with a sensitive data type;
   determine that the at least one data entry is a sensitive data entry.
31. The system of clause 21 wherein the redaction module is configured to: identify the at least one additional data entry as a sensitive data entry using sensitive text-based content.
32. The system of clause 21 wherein the redaction module is configured to provide a set of predetermined descriptor labels.
33. The system of clause 21 wherein the redaction module is configured to receive at least one user-defined descriptor label.
34. The system of clause 21 wherein the redaction module is configured to define for each sensitive data entry, a link between the sensitive data entry and a data type; and define a link between each data type and a descriptor label.
35. The system of clause 21 further comprising a form engine configured to provide a form and receive form field data values at the form; and define attributes for field objects, wherein the attributes comprise a sensitive data determination attribute and a caption attribute;
   and wherein the redaction module is further configured to:
   define a link between the form data value and a corresponding form object, wherein the field object is configured to define a form field, wherein the form field is configured to receive the form field data value;
   determine that the at least one data entry in the document is a sensitive data entry based on the sensitive data determination attribute of the corresponding form field object; and
   define the linked descriptor label using the caption attribute of the corresponding form field object.
36. The system of clause 21 wherein the user interface component is configured to provide the document using a mark up language, wherein the document comprises a mark up language attribute tag for each sensitive data entry in the document;
   and wherein the redaction module is configured to identify a sensitive data entry using the mark up language attribute tag in the document;
   and wherein the user interface component is configured to generate the redacted document using the mark up language attribute tag for the sensitive data entry.
37. A non-transitory computer-readable medium upon which a plurality of instructions are stored, the instructions for performing the steps of the method of clause.

## Claims

1. A method for redacting sensitive data entries, wherein the method is implemented on a processor having access to a memory in which instructions are stored, the instructions being executable to configure the processor to perform operations comprising:
receiving a command to generate a document, wherein the document comprises data entries, wherein each data entry comprises text-based content;
identifying at least one sensitive data entry in the document;
defining a link between each identified sensitive data entry and a descriptor label;
storing the link and the descriptor label in a repository; and
generating a redacted document by, for each identified sensitive data entry,
replacing the text-based content with the associated descriptor label.

2. The method of claim 1 further comprising:
receiving a command to redact at least one sensitive data entry in the document; and
generating the redacted document by, for each of the at least one sensitive data entries in the command to redact, replacing the text-based content with the associated descriptor label.

3. The method claim 1 or 2 further comprising:
receiving a single command to redact all sensitive data entries in the document;
identifying all sensitive data entries in the document; and
generating the redacted document by, for each sensitive data entry in the document, replacing the text-based content with the associated descriptor label.

4. The method of any one of claims 1 to 3 wherein the command to generate the document comprises a user identifier, and wherein at least one identified sensitive data entry in the document is identified based on the user identifier.

5. The method of any one of claims 1 to 4 further comprising receiving a command to export the document; and exporting the redacted document.

6. The method of claim 5 wherein the command to export comprises a recipient identifier, and wherein at least one identified sensitive data entry in the document is identified based on the recipient identifier

7. The method of claim 5 or 6 wherein exporting is selected from the group consisting of: printing, saving, transmitting and emailing.

8. The method of any one of claims 1 to 7 wherein each sensitive data entry is selectable, and wherein the method further comprises:
receiving a selected sensitive data entry;
receiving a descriptor label; and
defining a link between the selected sensitive data entry and the received descriptor label.

9. The method of any one of claims 1 to 7 wherein each sensitive data entry is selectable, and wherein the method further comprises:
receiving a selected sensitive data entry;
providing a set of descriptor labels, wherein each descriptor label is selectable;
receiving a selected descriptor label; and
defining a link between the selected sensitive data entry and the selected descriptor label.

10. The method of any one of claims 1 to 9 wherein determining whether the document contains one or more sensitive data entries comprises:
receiving sensitive text-based content;
determining whether a data entry in the document comprises the sensitive text-based content; and
upon determining that the data entry in the document comprises the sensitive text-based content, determining that the data entry is a sensitive data entry.

11. The method of any one of claims 1 to 10 wherein determining whether the document comprises one or more sensitive data entries comprises:
associating at least one data entry in the document with a data type;
defining at least one data type as a sensitive data type;
determining, for each of the at least one data entry in the document, whether the associated data type is a sensitive data type; and
upon determining that the associated data type is a sensitive data type,
determining that the data entry is a sensitive data entry.

12. The method of any one of claims 1 to 11 wherein the identified sensitive data entry comprises sensitive text-based content; wherein at least one additional data entry in the document comprises the sensitive text-based content; and wherein the method further comprises:
identifying the at least one additional data entry as a sensitive data entry using the sensitive text-based content;

13. The method of any one of claims 1 to 12 wherein at least one descriptor label is selected from a set of predetermined descriptor labels.

14. The method of any one of claims 1 to 13 wherein at least one descriptor label is a user-defined descriptor label; wherein the method further comprises receiving the user-defined descriptor label.

15. The method of any one of claims 1 to 14 wherein defining a link between each sensitive data entry and a descriptor label further comprises:
defining, for each sensitive data entry, a link between the sensitive data entry and a data type; and
defining a link between each data type and a descriptor label.

16. The method of any one of claims 1 to 15 wherein the at least one data entry in the document comprises a form field data value; and wherein the method further comprises:
defining a link between the form field data value and a corresponding form field object, wherein the field object is configured to define a form field, wherein the form field is configured to receive the form field data value;
defining attributes for the form field object, wherein the attributes comprise a sensitive data determination attribute and a caption attribute;
determining that the at least one data entry in the document is a sensitive data entry based on the sensitive data determination attribute of the corresponding form field object; and
defining the linked descriptor label using the caption attribute of the corresponding form field object.

17. The method of any one of claims 1 to 16 further comprising:
providing the document using a mark up language, wherein the document comprises a mark up language attribute tag for each sensitive data entry in the document;
identifying a sensitive data entry using the mark up language attribute tag in the document; and
generating the redacted document using the mark up language attribute tag for the sensitive data entry.
